# EUROPEAN PATENT SPECIFICATION

(11) **EP 1 098 719 B1**
(45) Date of publication and mention of the grant of the patent: **18.04.2007**
(21) Application number: 99934839.4
(22) Date of filing: 23.07.1999
(51) Int. Cl.: B06B 1/02, G01H 11/06

(54) **METHOD OF MANUFACTURING A CAPACITIVE ULTRASOUND TRANSDUCER**
VERFAHREN ZUR HERSTELLUNG EINES KAPAZITIVEN ULTRASCHALLWANDLERS
PROCEDE DE FABRICATION D'UN TRANSDUCTEUR CAPACITIF ULTRASONORE

(30) Priority: 23.07.1998 GB 9815992
(43) Date of publication of application: 16.05.2001
(73) Proprietor: QinetiQ Limited, London, SW1 6TD (GB)
(72) Inventor: NOBLE, Russell, Malvern, Worcestershire WR14 3PS (GB); BOZEAT, Robert John, Malvern, Worcestershire WR14 3PS (GB)
(86) International application number: PCT/GB1999/002217
(87) International publication number: WO 2000/005001

(56) References cited:
- US-A- 5 335 210
- PATENT ABSTRACTS OF JAPAN vol. 012, no. 177 (E-613), 25 May 1988 (1988-05-25) & JP 62 284600 A (AGENCY OF IND SCIENCE & TECHNOL), 10 December 1987 (1987-12-10)
- PATENT ABSTRACTS OF JAPAN vol. 011, no. 061 (E-483), 25 February 1987 (1987-02-25) & JP 61 220600 A (NEC CORP), 30 September 1986 (1986-09-30)
- PEDERSEN M ET AL: "A silicon condenser microphone with polyimide diaphragm and backplate" SENSORS AND ACTUATORS A, vol. 63, no. 2, 30 October 1997 (1997-10-30), page 97-104 XP004125898 ISSN: 0924-4247
- SCHEEPER P R ET AL: "FABRICATION OF SILICON CONDENSER MICROPHONES USING SINGLE WAFER TECHNOLOGY" JOURNAL OF MICROELECTROMECHANICAL SYSTEMS, vol. 1, no. 3, 1 September 1992 (1992-09-01), pages 147-154, XP000358599 ISSN: 1057-7157
- THIELEMANN C ET AL: "Capacitive silicon sensors for ultrasound" ACUSTICA - ACTA ACUSTICA, JULY-AUG. 1997, S. HIRZEL VERLAG, GERMANY, vol. 83, no. 4, pages 715-720, XP002121908 ISSN: 0001-7884
- MATTILA P ET AL: "BANDWIDTH CONTROL OF AN ELECTROSTATIC ULTRASONIC TRANSDUCER" SENSORS AND ACTUATORS A, vol. A45, no. 3, 1 December 1994 (1994-12-01), pages 203-208, XP000482630 ISSN: 0924-4247

## Description

This invention relates to micromechanical devices, in particular transducers, and especially to ultrasonic transducers. The term "transducer" is intended to cover any device which transmits energy from one system to another in the same or a different form. For example, acoustic or ultrasonic transducers or accelerometers which convert acoustic energy, ultrasonic energy or acceleration force into movement of a deflectable membrane or plate are considered to fall within the definition, and also actuators which convert electrical energy into movement. However, ultrasonic transducers - detectors and emitters, and especially arrays of transducers, are an area of special interest and form their own class of transducers. The invention also relates to devices (including sensors and actuators) fabricated using what can be described as micromachining techniques.

An example of micromechanical device is an ultrasonic transducer. It is known to have an ultrasonic transducer that comprises a membrane, with a formed electrode, over a resonant cavity and a fixed substrate to which a second electrode is formed. The membrane is typically formed from a preformed planar sheet of semiconductor material (such as silicon dioxide, silicon nitride, and polysilicon) applied to the substrate.

The membrane deflects upon contact with an incident ultrasonic wave. Movement of the membrane varies the capacitance between the electrodes which can be used to detect incident ultrasonic waves.

Ultrasonic transducers with a semiconductor membrane operate well, but with high quality factor (high Q), due to the relatively high intrinsic rigidity associated with the material used for the membrane.

There are circumstances where a high Q is not desirable. For example, during medical imaging, guidance and positioning, and structural inspection (e.g. of articles) a high bandwidth, low Q, ultrasonic detector is required. Ultrasonic high Q, low bandwidth, detectors may not be suitable for this purpose.

It has been reported in the paper by D.W. Schnidel and D.A. Hutchins, IEEE Trans. on Ultrasonics Ferroelectrics and Frequency Control vol. 42, number 1, January 1995, that if transducers are fabricated by adhering a PCB polymer sheet as a membrane onto a substrate a low Q, high bandwidth, transducer may be achievable. This process for manufacturing such transducers has a low yield and does not give reproducible results. It is difficult to produce many transducers which have similar or identical properties. Bonding the sheet of polymer to the silicon substrate is not easy. Obtaining a uniform bond, with no areas of poor bonding can be difficult. Any irregularities in the bonding of the sheet of polymer to the silicon substrate causes variations in the performance of the device. Furthermore, the bonding process is not a step familiar to those skilled in the production of silicon integrated devices and it is therefore not a simple matter to convert existing processing facilities for use in producing such devices. It is not repeatable, controllable or suitable for mass production. It is not compatible with standard IC fabrication techniques, for example CMOS.

Ultrasonic transducers having polymer membranes are known from JP-A-62 284 600 and JP-A-61 220 600.

According to a first aspect, the invention provides an ultrasound detector or emitter apparatus comprising a substrate having a movable polymer membrane attached to it, and a single chamber defined between the membrane and the substrate, the membrane and the substrate in the region of the chamber each having a respective electrical contact pad, the arrangement being such that relative movement between the contact pads is either indicative of incident ultrasound, or causes ultrasound to be generated characterised in that the movable membrane is not formed in a single plane.

According to another aspect the invention provides a method of producing an ultrasonic transducer comprising applying a sacrificial material to a substrate, applying a polymer coating over at least part of the sacrificial material and the substrate, and removing at least part of the sacrificial material to leave a portion of the polymer coating defining a movable member wherein the member defines a part of a single cavity, and in which the polymer is applied in a mobile state characterised in that the sacrificial material assists in defining a non-flat shape of movable polymer membrane and the sacrificial material is removed after the shape of the movable polymer membrane has been established.

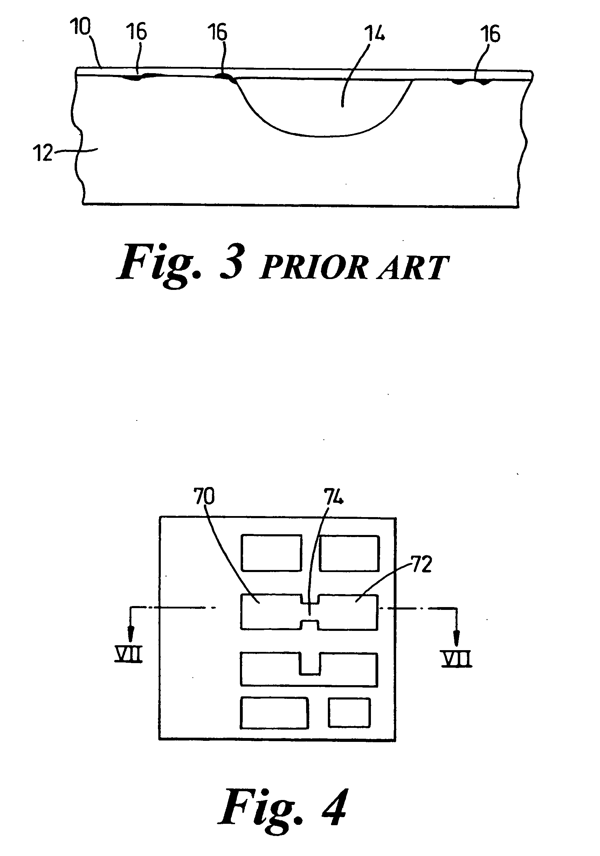

A search has also found WO97/19572 which discloses a transducer for use as a microphone having a planar polymer flexible membrane overlying a space formed by removing a sacrificial material. The overall direction of the document is to use a thick rigid membrane that does not move and a thinner movable membrane to define a chamber. The document directs away from using a SiO₂ insulating layer on a silicon substrate (see p. 2, final paragraph), instead a different insulating layer (e.g. of polyimide) is used. The document is primarily concerned with its Figures 2 and 3 may also be suitable for gross movements such as a tactile, touch, or pressure sensor and that the sensor of Figure 2 (but not Figure 3) may be suitable as an acceleration sensor.

It will be noted that WO97/19572 is not concerned with ultrasonic transducers, or ways of detecting ultrasound, or ultrasonic imaging, ultrasonic testing of structures using structurally transmitted ultrasound: WO97/19572 does not disclose producing an array of transducers (ultrasound or other).

Preferably the polymer coating is applied over substantially the whole of the sacrificial material. Preferably substantially the whole of the sacrificial material is removed. The movable member may be deflectable and/or deformable. The movable member may comprise a membrane.

The movable member preferably defines a membrane of an ultrasonic transducer. Alternatively, it may define a displaceable mass (for example the plate of an accelerometer).

Thus, in comparison with the arrangement of the paper by Schnidel et al where a substantially rigid sheet of material is glued to a substrate to form a membrane, the membrane material of the present invention is highly mobile (e.g. a liquid) in the application state. The member, or membrane, can therefore adhere itself to the substrate without the need for a separate adhesive as the polymer is cured following application. WO97/19572 is not concerned with ultrasonic transducers.

The method thus comprises providing a layer of sacrificial material on the substrate, using the sacrificial material to assist in defining the shape of the cavity/movable member, and removing the sacrificial material after the shape of the cavity defined by the polymer member and the substrate has been established. This may be used in the fabrication of an ultrasound transducer, or in an array of transducers or a common semiconductor substrate (e.g. silicon).

The method may comprise dissolving away, or otherwise removing, some or all of the sacrificial material within the cavity (or cavities in the case of an array/where there is more than one cavity) after the polymer material has been, at least partially, cured. Preferably the sacrificial material is removed after the polymer material has been substantially fully cured.

The method may therefore comprise applying a layer of sacrificial material over a substrate, which is preferably a semiconductor material, such as silicon or galium-arsenide and forming the sacrificial layer to create a profiled terrain. Projections of sacrificial material may be created extending away from the substrate or alternatively recesses may be provided in the substrate which are filled with sacrificial material. Preferably regions of the sacrificial layer are completely removed prior to the application of the polymer material, so that the polymer material directly contacts the substrate (e.g. silicon, or silicon covered with an insulator, such as SiO₂) in parts, and overlies the sacrificial material in other parts, where it is applied. Islands of sacrificial material may be created.

The sacrificial layer may be shaped using surface micromachining techniques. For example it may be shaped, using photolithographic mask techniques - e.g. optical, x-ray, electron beam techniques in conjunction with etching techniques, (e.g. wet or dry etching), or by other suitable direct removal techniques e.g. laser or focused ion beam.

Surface micromachining techniques are well known in the production of semiconductor devices/in IC production. Much of the equipment and techniques used to perform surface micromachining exists and is based on that used for fabricating microelectronics (e.g. CMOS technology). It is preferable to produce the transducer using standard micromachining technologies and as many common materials as possible within existing commercial semiconductor device production facilities. The process proposed is a low temperature process (approximately 400°C or less) and is compatible with existing electronics technology, e.g. CMOS.

Surface micromachining enables us to produce a polymer membrane ultrasonic transducer in a more controllable way, and with a higher yield than other techniques for producing ultrasonic transducers. Furthermore, it allows us to produce an array of transducers (which may be ultrasonic transducers) on a chip/silicon (or other substrate). Furthermore, the polymer membrane ultrasonic transducers (or arrays) produced are more compatible with CMOS and bipolar mass production of semiconductor devices than are hand-made transducers with sheets of PCB bonded to a substrate. The properties of the polymer member including mass, thickness, size and shape, and stiffness can all be readily controlled. This leads to control of subsequent transducer properties.

The method may comprise the following additional steps once the dimensions of the substrate, cavity, and membrane have been decided. Firstly, a bottom contact pad material (e.g. metal) may be deposited onto the substrate (e.g. SiO₂ thin layer over silicon). Next, a bottom contact pad mask may be used to define an area for defining the bottom contact pad of the transducer (e.g. ultrasonic transducer) and the material surrounding the mask removed using an etching process (or other removal process). The bottom contact pad material may be applied by sputtering, or other particle deposition processes. The bottom pad may form one electrode of a capacitor and may be metal. Alternatively, a bottom contact pad material may be deposited through a mask.

A pre-step of electrically insulating the surface of the semi-conductor surface may be performed, for example using a layer of silicon dioxide for silicon substrates if such a layer is not already present.

Secondly, the method may comprise the further steps of applying a sacrificial layer over the bottom electrode and using a cavity mask to define the area that will be the cavity of the transducer and removing material from the sacrificial layer not covered by the cavity mask.

Thirdly, a polymer membrane may be applied, e.g. spin coated, over both the sacrificial material and optionally the area of substrate immediately surrounding the sacrificial material to form the shape of the membrane. The membrane material may then be cured.

In a fourth step, the method may comprise applying a top contact pad material onto the surface of the membrane (preferably the upper surface) and using a top contact pad mask to define the area of the electrode/top contact pad before removing material outside the masked area to leave only material defining the top contact pad. The top contact pad material may be sputtered on, or otherwise deposited, and removed by etching. Alternatively, a top pad may be deposited through a mask.

Finally, the method may comprise using an etch hole mask to define etch holes, and removing material to produce the etch holes to enable sacrificial material to be etched out, via the etch holes. This leaves a substantially empty (apart from the bottom contact) cavity defining the sensor. The etch holes also enable the pressure on either side of the membrane to be equalised. Thus, the etch holes penetrate completely through the membrane.

As a variation to this fabrication, using similar sacrificial and membrane layers, a transducer could easily be formed in which the cavity comprises a recess in the substrate surface with the membrane on top. This may be used to realise a transducer which is substantially flat on the substrate surface.

The production process may be a four mask process.

It will be appreciated that it is envisaged to apply a polymer material over a substrate when the polymer material is in a mobile state, ensure that the polymer material has the desired shape and thickness, cure the polymer material, and leave the cured polymer material as part of the finished micromechanical device.

The method may comprise producing an array of transducers on the same substrate, and/or ensuring that there are transducers which respond to different frequencies. The method may further comprise linking the cavities of selected transducers to modify the performance of an array of transducers. This can be achieved by micro-machining channels into the insulating layer between where the subsequent resonant cavities would be formed later in the sequence. This could be done using lithographic masking and etching. An extra sacrificial layer could be introduced on top of these channels which may overlap the edges of the channels and the regions where the cavities would be formed. The extra sacrificial material in the channels could then automatically be removed during the final etching of the material defining the cavities.

The method may comprise providing an integrated semiconductor device having the transducer and having signal processing means provided on the same substrate. The signal processing means may be defined in the semiconductor device in a first stage, and the micromechanical device or transducer created in a subsequent stage. The formation of the transducer does not damage the circuitry of the signal processing means as it is formed using a low temperature process.

The production of the integrated semiconductor device that incorporates a polymer membrane ultrasonic transducer is greatly facilitated by making the transducer using surface micromachining techniques that are CMOS compatible. Both etching and spin coating are well developed techniques in semiconductor fabrication, and so commercial plants can be readily adapted to produce the transducers of the present invention. The spin coating is a low temperature technique, allowing membranes to be applied to substrates which include pre-defined electronic circuits without damaging the circuits.

By getting the processor close to the sensor we reduce noise and can detect lower signals than would otherwise be possible. Since we may wish to detect very small changes in capacitance in the transducer, being able to have integrated electronics can be significant. Integration of the transducer with the control/processing electronics enables us to provide very small micromechanical devices.

The polymer material may be a polyimide, and is preferably PIQ™ (available from Hitachi). The polymer material may be applied to the substrate, or to sacrificial material if it is provided as a liquid, and is spread out over the surface of the substrate or sacrificial material to coat it. The polymer material may be spun coated onto the substrate and/or sacrificial material, centrifugal forces being used to control the thickness of the coating. A knowledge of the physical properties of the material in its mobile viscous (liquid) state can be used to control the processing conditions, e.g. spin coating.

The bandwidth of the transducer operating as an ultrasonic transducer, for example in structural application, may be of the order of several kHz to several Mhz, preferably in the range of 100kHz-10 Mhz, or 1-8 Mhz, or 2-6 Mhz, or at least 3, 4 or 5 Mhz. The Q factor may be low compared to devices using other semiconductor membranes. Of course, for other applications the bandwidth could be very different, e.g. from Hz to Mhz.

According to a further aspect of the invention an integrated semiconductor device is provided having an ultrasonic transducer in accordance with the invention provided on a semiconductor substrate and a signal processor or signal modifier provided on the same semiconductor substrate, integrating the processor and the transducer in the same device.

According to a fifth aspect of the invention an integrated semiconductor device is provided having an ultrasonic transducer in accordance with the second aspect of the invention provided on a semiconductor substrate and a signal processor or signal modifier provided on the same semiconductor substrate, integrating the processor and the transducer in the same device.

According to a sixth aspect, the invention provides an integrated semiconductor device having a plurality of ultrasonic polymer membrane transducers provided as an array.

The array may have associated with it, on the same semiconductor substrate, signal processing or signal conditioning electronics.

According to a seventh aspect, the invention provides a structure comprising a semiconductor substrate having a polymer member defined thereon, in which the polymer member is applied to the substrate in a mobile state.

The member may be movable or non-movable. The member may be rigid, and may define part of a cavity. It may define a membrane or cover over the cavity, or define a lever or projection extending from the substrate.

An embodiment of the invention will now be described by way of example only with reference to the accompanying drawings, in which:-
**Figure 1** schematically shows an ultrasonic transducer in accordance with the invention;
**Figure 2** shows a plan view of the transducer of Figure 1;
**Figure 3** shows schematically a prior art transducer;
**Figure 4** schematically shows an integrated array of transducers, with linking of chambers;
**Figure 5** is a cross-section on line VII-VII of Figure 4; and
**Figure 6** shows an ultrasonic transducer in which a chamber is provided in the substrate and a polymer membrane extends over the recess.

Figure 3 illustrates one prior art arrangement for producing ultrasonic transducers comprising a substantially rigid, planar polymer membrane attached to a silicon substrate. A sheet of the membrane material is taken and attached to a silicon substrate material 12, which has had formed in it a recess 14. The sheet of membrane material is, typically, glued onto the silicon substrate. There can be problems adhering the sheet of material properly to the silicon substrate, for example areas where poor bonding/no bonding at all has taken place are schematically illustrated in Figure 3 as dark areas 16. These will influence the operational performance of the transducer. Since the imperfections in bonding are not predictable, each transducer that is made will perform slightly differently. It is very difficult to achieve control of this type of response. Furthermore, if there are too many poor areas of bonding, or poor areas of bonding too close to the recess 14 (e.g. at its peripheral edge) then the transducer may not operate properly at all. The prior art production of ultrasonic transducers with polymer membranes has a high failure rate, and a low yield.

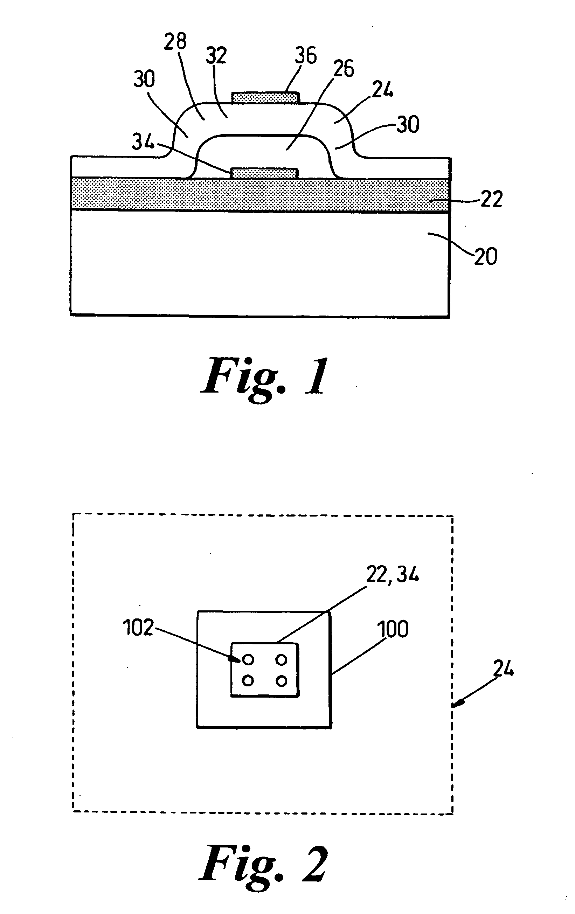

Figure 1 schematically illustrates a new ultrasonic transducer produced using the present invention. A silicon substrate 20 has a dielectric coating 22 (and in this example the dielectric is silicon dioxide), and a polymer membrane 24 (in this example a membrane made of PIQ™) extends over the dielectric 22 and defines a cavity 26 between a raised part of the membrane 28 and the dielectric/silicon substrate. Air is typically provided in the cavity 26, but some other contents may be provided for some circumstances.

The polymer membrane defined in the raised region 28 has side wall regions 30 which extend away from the dielectric/silicon substrate, and a top wall region 32 which extends generally parallel to the substrate. A metal bottom contact pad 34 is provided on the dielectric 22 in the cavity 26, and a top contact pad 36 is provided on top of the raised portion 28 of the polymer membrane.

The operating principle of the transducer is that the raised portion 28 of the polymer membrane is deflected by an incident ultrasound wave and that deflection results in a change in the capacitance between the top contact pad 36 and the bottom contact pad 34 as the top contact pad 36 moves relative to the bottom contact pad 34. The change in capacitance is measured, and this is used to derive the output of the ultrasonic transducer.

To manufacture the transducer of Figure 1 silicon surface micromachining technology is used.

A silicon substrate is surface cleaned using automated water cleaning equipment and then thermally oxidised in conventional semiconductor furnace equipment. An insulating layer of silicon dioxide is formed, approximately 600 nm (6000Å (angstroms)) in thickness.

The bottom (fixed) electrode is formed on the silicon dioxide by sputter depositing a combination of different metal layers to form a sandwich structure. A thickness of approximately 400 nm (4000Å (angstroms)) of titanium-tungsten (TiW) and aluminium silicon (AlSi) layers are employed. TiW is employed as the principle bottom electrode material. This is because AlSi is employed as the sacrificial layer for the resonant cavity, and this sacrificial layer must later be removed with the bottom electrode remaining intact. An AlSi layer as part of the bottom electrode structure serves to protect the TiW during the polyimide etch which occurs later, and during which any unprotected TiW material would also be etched. The bottom contact pad is defined in the deposited metal layers using an (optical) lithographic process and conventional photoresist mask, followed by a reactive ion etch (RIE), based on a combination of chemistry's; CF₄, O₂ and BCl₃, Cl₂, CHF₃ (for TiW and AlSi respectively).

Thus the bottom electrical contact pad is made from layers of material, and has a layer which protects lower layers from the etch process used to remove the sacrificial material that defines the cavity, at least some of the lower layers of the pad being otherwise etchable by the etchant.

The sacrificial layer comprises Aluminium Silicon (AlSi) and is introduced over the regions of bottom metal contact pad and oxidised silicon surface. Aluminium silicon (AlSi) is sputter deposited to a thickness which will subsequently set the resonant cavity thickness. This thickness is dependent on the required ultrasonic performance of the device but typically may be of the order of a few micrometers (10⁻⁶ m). The size and shape of the resonant cavity is defined in the sacrificial layer using an (optical) lithographic process and conventional photoresist mask, followed by a reactive ion etch (RIE), based on a BCl₃, Cl₂, CHF₃ chemistry.

The polymer membrane is next formed over the entire substrate, covering the areas of sacrificial material and contacting the substrate at substantially all sides of the sacrificial material. This is done using automated equipment by dispensing polyimide (Hitachi PIQ™), in liquid form, onto the substrate and spinning the substrate at high speeds to produce a known film thickness, typically 1 to 3µm, and preferably not more than about 5 µm, which is governed by the ultrasonic properties required for the transducer. The membrane must ideally be a highly uniform film, with specific mechanical properties (intrinsic stress), and with absolute minimal defects. Adequate preparation and cleaning of the substrate surface should be performed prior to this step, and after previous lithographic masking and etching, to ensure good adhesion between the polyimide and substrate materials. The polyimide is then 'cured' with a thermal treatment to harden the film, but also importantly for this transducer application to control its mechanical properties i.e. ideally to set a low intrinsic tensile stress. This is done at approximately 370°C in conventional semiconductor furnace equipment.

The top electrode, which forms the movable plate of the variable capacitor attached to the deflectable membrane, is next introduced. This is done by sputter depositing a metal layer of titanium-tungsten (TiW) on top of the polyimide membrane. This process is critical in that the metal deposition conditions must be carefully controlled in order to minimise any effect on the mechanical properties of the membrane, which ultimately govern the ultrasonic properties. A substrate pre-bake is employed to minimise 'outgassing' from the polyimide film during deposition. Chamber conditions in the sputtering equipment must be controlled such as pressure, as must the deposition temperature. A typical thickness would be approximately 100 nm (1000Å) to 200 nm (2000Å) of TiW material, deposited at approximately 250°C or higher. The top contact pad is defined in the deposited metal layer using an (optical) lithographic process and conventional photoresist mask, followed by a reactive ion etch (RIE), based on a CH₄, O₂ chemistry.

The next stage is to introduce the etch holes through the top metal electrode and membrane materials down to the top of the sacrificial layer. Etch holes are defined using an (optical) lithographic process and conventional photoresist mask, followed by a combination reactive ion etch (RIE) of the metal and polyimide materials. The etch holes must allow sufficient access for an etchant material to be able to remove the sacrificial layer and form the resonant cavity. However, since the mass and stiffness of the membrane contribute to the ultrasonic properties of the transducer, holes introduced through the membrane should be designed to have a minimum effect. Hence a number of very small etch holes (approximately 3µm diameter) with a reasonable spacing (10's of µm) are employed. The RIE steps utilise a combination of chemistry's; CF₄, O₂ and CHF₃, O₂ (for TiW and PIQ respectively).

The final stage is to use a suitable etchant material to remove the sacrificial layer (AISi) from under the membrane. The substrate is immersed in a proprietary metal etch solution (ISOFORM) for a time sufficient to permit the etchant to penetrate the etch holes, and laterally etch the sacrificial AlSi between the etch holes, and completely clear (or substantially clear) the resonant cavity. Substrates are then rinsed and dried.

It will be appreciated that in comparison with the prior art shown in Figure 3, the membrane 24 is profiled: it extends away from the surface of the silicon substrate (it could also be flat to the surface if the cavity is recessed). Furthermore, the polymer membrane is applied as a liquid, and this improves the bonding - it is different from applying a preformed sheet of material to the silicon substrate. The use of the sacrificial material to define the cavity positively (by having contact between the polymer membrane and the sacrificial material whereupon the membrane is setting/curing so as to give support to the membrane) gives better control of the shape and size of the cavity than the prior art (in which the sheet of polymer may sag into the recess 14 depending upon the conditions experienced).

In comparison with the prior art of WO97/19572 the present invention allows arrays to be made with the substrate providing support, and enables ultrasonic detectors and arrays to be made.

The surface micromachining techniques used to produce the transducer of Figure 1 could also be used to link resonant cavities of transducers in an array.

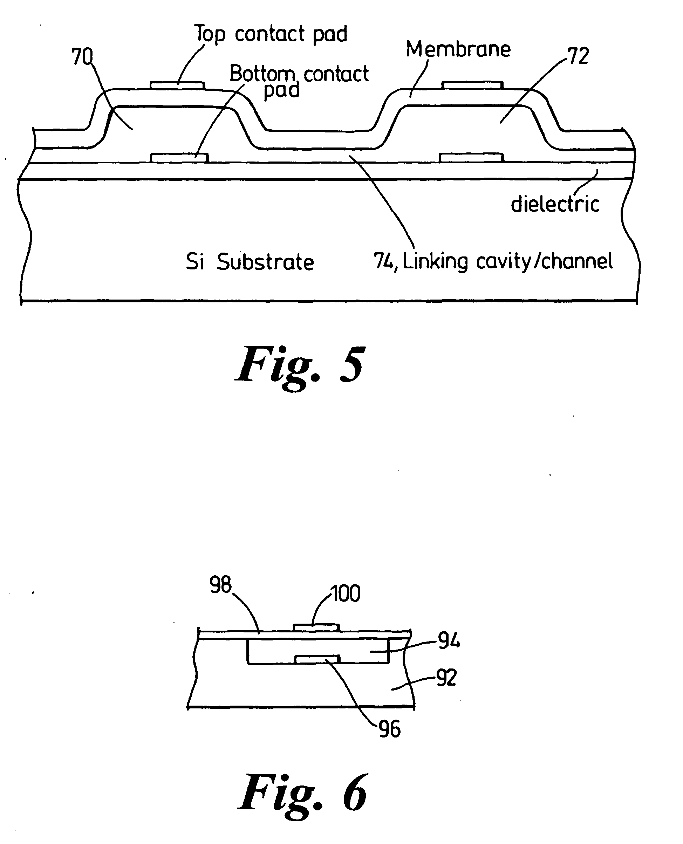

Figures 4 and 5 show an arrangement where two adjacent chambers 70,72 have been linked by a passage 74. In Figure 5, passage 74 is shown as being a central passage forming a symmetrical "double" chamber, but the arrangement could be non-symmetrical (for example the lower two chambers of the device of Figure 5 could be linked by a passageway that is non-symmetrically disposed relative to the centre-lines of the two chambers). The chambers could have different shapes, or areas, or depths, or any combination of shape/depth/area.

It will be appreciated that the polymer material used to make the movable member of the transducer needs to have certain mechanical and chemical properties, but it is unlikely that PIQ™ will be the only material suitable. The membrane needs to be able to bond well to the substrate/silicon dioxide dielectric. It needs to be able to have the metal top contact pad sputter-deposited on it, and needs to be able to withstand the conditions experienced during that sputtering operation (e.g. it needs to be able to withstand temperatures of a few hundred degrees centigrade). The polymer material should not have too high an intrinsic stress or when the sacrificial material is removed it will deform to a shape that is not carefully defined, and thereby make the production of ultrasonic transducers less predictable. The polymer material needs to be self-supporting when cured, again so as to control its shape. The polymer material needs to be able to withstand attack by the etching substances used in the removal of the sacrificial material. The polymer material needs to be an electronically compatible material - having little or no impurities (such as gold) likely to influence the electronic operation of the device. We believe that polyimide materials are best suited to forming the membrane, although we do not wish to be restricted to these. The materials traditionally used for photo-resist during CMOS manufacturing techniques may be suitable as membranes, and the techniques, and equipment developed for applying photo-resist may be readily adapted to application of the membrane material.

It will be noted that in the embodiments described TiW and TiW/AlSi are used for the upper and lower contact pads. It is believed that it is best to use materials that are compatible with I.C. fabrication techniques, such as CMOS. Gold is not considered to be a compatible material since it can be a contaminant and interfere with the proper operation of electronic circuitry that is embedded in a chip. Furthermore in addition to contaminating the chip it can contaminate the manufacturing equipment and then contaminate subsequent chips made on the equipment. Since the sacrificial material is AlSi, having AlSi in the bottom contact pad adds no new elements.

It may be desired to make arrays of transducer elements with their resonant cavities interconnected with sub-channels. It may be desirable to use an additional sacrificial layer (preferably metal based). For example, we could make discrete sacrificial layer-material raised portions, such as would be used to form the array shown in Figure 4, and then create "bridges" of further sacrificial material between the original arrays, at the locations wanted, by applying another layer of sacrificial material and then removing it in areas that are not for bridges.

It is envisaged that the transducers, most preferably ultrasonic transducers, will have applications in structural monitoring, medical imaging, guidance and positioning apparatus. Arrays of transducers may have especial applications, for example in at least some of the fields mentioned. Protection is sought for such apparatus incorporating a transducer (or an array of transducers) in accordance with the invention. Actuators and displacement sensors are also envisaged.

Since various transduction mechanisms govern the subsequent device performance (for example the Q) in ultrasonic devices, surface micromachining permits control over these different mechanisms by controlling the device structure parameters (for example the membrane mass, the stiffness, the size of cavity, and the ability to link cavities together in an array).

Of course, it will be readily understood that in one aspect the present invention lies in the production of a transducer of the kind in which the membrane (preferably a single membrane) is applied in a mobile state (preferably contacting a substrate such as silicon, or substrate insulator such as SiO₂) whilst supported by a sacrificial material, the membrane then being cured and the sacrificial material being removed. A transducer having a single micromachined polymer membrane is seen as being new and advantageous. The cavity defined by the membrane and the substrate may protrude above the surface of the substrate (as shown in the accompanying drawings). Alternatively, a hole or recess could be formed in the substrate which is filled (wholly or partially) with sacrificial material, and which may support the membrane during manufacture. The word cavity is not intended to be restricted to meaning a be fully enclosed area covered by the membrane, but includes a partially closed cavity, or a cavity defined by a membrane "bridge" over a void.

Also, the skilled man would readily understand that the material need not be in a fully liquid state when applied, but could be in any form whereby it will be deformed under its own weight or under gravity (due to, for example, spin coating) to take up a desired form before being cured (by temperature or any other process) to take on a permanent form.

The polymer member or structure applied in mobile form need not necessarily be movable. For example it could protect a structure beneath it, or could form a window for the passage of a variety of measurands. The polymer member or structure could be a mechanical structure defining part or all of a desired formation (for example it could comprise a cover, which may define in part one or more chambers or channels).

Figure 6 which represents prior art shows a silicon chip or substrate 92 having a recess 94 in which a bottom contact 96 is provided, and a polymer cover or membrane 98 bonded to the chip and overlying the chamber, with top contact 100 provided on it. The membrane 98 is generally in the plane of the surface of the chip 92.

A transducer may have a movable membrane that is of the order of 10 µm x 10 µm, or of the order of thousands of µm x thousands of µm, or any size in between. An array may have a size that is a few transducers x a few transducers, or tens x tens, or 100's x 100's, or even 1000's x 1000's.

The pads shown do not extend over the whole area of the movable polymer membrane.

Preferably the semiconductor (e.g. Silicon) substrate has a flat, planar, back surface.

It will be appreciated that the arrangement of the present invention is simpler to manufacture than that of WO97/19572.

It is envisaged that the ultrasound detectors may detect ultrasound in air, ultrasound in structures, or even be immersed.

## Claims

1. Ultrasound detector or emitter apparatus comprising a substrate (20) having a movable polymer membrane (24) attached to it, and a single chamber (26) defined between the membrane (24) and the substrate (20), the membrane (24) and the substrate (20) in the region of the chamber (26) each having a respective electrical contact pad (34,36), the arrangement being such that relative movement between the contact pads (34,36) is either indicative of incident ultrasound, or causes ultrasound to be generated **characterised in that** the movable membrane (24) is not formed in a single plane.

2. Apparatus according to claim 1 in which the substrate (20) is a semiconductor.

3. Apparatus according to claim 2 in which electronic circuitry is provided in the semiconductor substrate (20) either (i) to read out signals from the membrane and substrate pads, or (ii) to drive the pad on the movable polymer membrane, or both (i) and (ii).

4. Apparatus according to any preceding claim in which there is only a single membrane (24) or layer of polymer material.

5. Apparatus according to any preceding claim that is fabricated using an IC compatible process, including using IC compatible metal electrode materials.

6. Apparatus according to any preceding claim in which the pads (34,36) have no CMOS incompatible materials, such as gold.

7. Apparatus according to any preceding claim which comprises structural assessing apparatus adapted to test a structure for damage.

8. Apparatus according to any preceding claim which comprises a membrane (24) of no more than 3 µm thickness extending over the chamber (26).

9. Apparatus according to any preceding claim in which the movable membrane (24), which moves in use in response to incident ultrasound or which generates ultrasound, has a central portion (28) and at least one side portion which is not in the general plane of the central portion (28).

10. Apparatus according to any preceding claim that has a bandwidth of at least 1 MHz (6dB bandwidth).

11. An integrated semiconductor device having apparatus in accordance with any one of claims 1 to 10 provided on a semiconductor substrate and a signal processor or signal modifier provided on the same semiconductor substrate, integrating the processor and the transducer in the same device.

12. A method of producing an ultrasonic transducer comprising applying a sacrificial material to a substrate, applying a polymer coating over at least part of the sacrificial material and the substrate, and removing at least part of the sacrificial material to leave a portion of the polymer coating defining a movable member wherein the member defines a part of a single cavity, and in which the polymer is applied in a mobile state **characterised in that** the sacrificial material assists in defining a non-flat shape of a movable polymer membrane, and the sacrificial material is removed after the shape of the movable polymer member has been established.

13. A method according to Claim 12 in which the polymer coating which defines the movable member is applied over substantially the whole of the sacrificial material and contacts the substrate around the cavity.

14. A method according to Claim 12 or Claim 13 in which the polymer coating is applied at a thickness so as to produce a movable member that is of the order of 2 µm thick, or less.

15. A method according to any one of claims 12 to 14 in which the substrate is a semiconductor material.

16. A method according to any one of claims 12 to 15 comprising applying a top contact pad material onto the top of the membrane, and etching the material through a mask to define a top contact pad.

17. A method according to any one of claims 12 to 16 which further comprises providing an integrated semiconductor device having the transducer and having signal processing means provided on the same substrate.

## Patentansprüche

1. Ultraschalldetektor- oder Ultraschallsendegerät, welches umfaßt:
ein Substrat (20), welches eine daran befestigte bewegliche Polymermembran (24) aufweist, und eine einzige Kammer (26), die zwischen der Membran (24) und dem Substrat (20) vorgegeben ist, wobei die Membran (24) und das Substrat (20) im Bereich der Kammer (26) jeweils ein entsprechendes elektrisches Kontaktfeld (34, 36) aufweisen und die Anordnung derart ist, dass die relative Bewegung zwischen den Kontaktfeldern (34, 36) entweder einen auftreffenden Ultraschall anzeigt oder bewirkt, dass ein Ultraschall erzeugt wird,
**dadurch gekennzeichnet, daß** die bewegliche Membran (24) nicht in einer einzigen Ebene ausgebildet ist.

2. Gerät nach Anspruch 1, bei dem das Substrat (20) ein Halbleiter ist.

3. Gerät nach Anspruch 2, bei dem die elektronische Schaltungsanordnung im Halbleitersubstrat (20) vorgesehen ist und entweder (i) Signale aus der Membran und den Substratfeldern ausliest oder (ii) das Feld auf der beweglichen Membran antreibt oder beides, (i) und (ii).

4. Gerät nach einem der vorhergehenden Ansprüche, welches nur eine einzige Membran (24) oder eine Schicht aus Polymermaterial aufweist.

5. Gerät nach einem der vorhergehenden Ansprüche, welches unter Anwendung eines für integrierte Schaltungen (IC) kompatiblen Verfahrens, beispielsweise unter Verwendung von IC-kompatiblen Metallelektrodenmaterialien, hergestellt ist.

6. Gerät nach einem der vorhergehenden Ansprüche, bei dem die Felder (34, 36) keine CMOS-kompatiblen Materialien, wie z.B. Gold, aufweisen.

7. Gerät nach einem der vorhergehenden Ansprüche, welches ein Aufbaubewertungsgerät umfaßt, das so ausgeführt ist, dass es einen Aufbau auf Fehler testet.

8. Gerät nach einem der vorhergehenden Ansprüche, welches eine Membran (24) einer Dicke von nicht mehr als 3 Mikrometern umfaßt, die sich über die Kammer (26) erstreckt.

9. Gerät nach einem der vorhergehenden Ansprüche, bei dem die bewegliche Membran (24), die sich im Betrieb unter Ansprechen auf einen auftreffenden Ultraschall bewegt oder Ultraschall erzeugt, einen zentralen Abschnitt (28) und mindestens einen Seitenabschnitt aufweist, welcher sich nicht in der allgemeinen Ebene des zentralen Abschnitts (28) befindet.

10. Gerät nach einem der vorhergehenden Ansprüche, welches eine Bandbreite von mindestens 1 MHz (6 dB Bandbreite) aufweist.

11. Integrierte Halbleitervorrichtung, die ein Gerät gemäß einem der Ansprüche 1 bis 10 aufweist, das auf einem Halbleitersubstrat und einem Signalprozessor oder einem Signalwandler angeordnet ist, die auf demselben Halbleitersubstrat vorgesehen sind und Prozessor und Wandler in demselben Gerät integriert sind.

12. Verfahren zur Erzeugung eines Ultraschallwandlers, welches die Schritte umfaßt:
- Aufbringen eines Opfermaterials auf ein Substrat,
- Aufbringen einer Polymerbeschichtung auf mindestens einen Teil des Opfermaterials und das Substrat und
- Entfernung mindestens eines Teils des Opfermaterials, sodaß ein Abschnitt der Polymerbeschichtung übriggelassen wird, die ein bewegliches Element vorgibt, wobei das Element einen Teil eines einzigen Hohlraums vorgibt, und das Polymer in einem mobilen Zustand aufgebracht wird,
**dadurch gekennzeichnet, daß** das Opfermaterial das Vorgeben einer nicht ebenen Form einer beweglichen Polymermembran unterstützt und das Opfermaterial entfernt wird, nachdem sich die Form des beweglichen Polymerelements gebildet hat.

13. Verfahren nach Anspruch 12, bei dem die Polymerbeschichtung, die das bewegliche Polymerelement vorgibt, im wesentlichen über die Gesamtheit des Opfermaterials aufgebracht wird und mit dem Substrat um den Hohlraum herum in Berührung kommt.

14. Verfahren nach Anspruch 12 oder 13, bei dem die Polymerbeschichtung mit einer Dicke aufgebracht wird, damit ein bewegliches Element in der Größenordnung einer Dicke von 2 Mikrometern oder darunter erzeugt wird.

15. Verfahren nach einem der Ansprüche 12 bis 14, bei dem das Substrat ein Halbleitermaterial ist.

16. Verfahren nach einem der Ansprüche 12 bis 15, welches die Schritte umfaßt:
- Aufbringen eines obersten Kontaktfeldmaterials oben auf die Membran und
- Ätzen des Materials durch eine Maske zum Vorgeben eines obersten Kontaktfeldes.

17. Verfahren nach einem der Ansprüche 12 bis 16, welches ferner den Schritt des Vorsehens einer integrierten Halbleitervorrichtung umfaßt, welche den Wandler und eine Signalverarbeitungseinrichtung enthält, die auf demselben Substrat vorgesehen sind.

## Revendications

1. Appareil détecteur ou émetteur ultrasonore comprenant un substrat (20) ayant une membrane polymère mobile (24) fixée à celui-ci, et une chambre unique (26) définie entre la membrane (24) et le substrat (20), la membrane (24) et le substrat (20) dans la région de la chambre (26) ayant chacun un plot de contact électrique respectif (34, 36), l'agencement étant tel que le mouvement relatif entre les plots de contact (34, 36) est soit indicatif des ultrasons incidents, soit provoque la génération d'ultrasons, **caractérisé en ce que** la membrane mobile (24) n'est pas formée dans un plan unique.

2. Appareil selon la revendication 1, dans lequel le substrat (20) est un semi-conducteur.

3. Appareil selon la revendication 2, dans lequel le circuit électronique est prévu dans le substrat semi-conducteur (20) soit (i) pour lire les signaux des plots de la membrane et du substrat, soit (ii) pour commander le plot sur la membrane polymère mobile, soit pour (i) et (ii) à la fois.

4. Appareil selon l'une quelconque des revendications précédentes, dans lequel il n'existe qu'une seule membrane (24) ou couche de matière polymère.

5. Appareil selon l'une quelconque des revendications précédentes qui est fabriqué en utilisant un processus compatible CI, comprenant l'utilisation de matériaux d'électrodes métalliques compatibles CI.

6. Appareil selon l'une quelconque des revendications précédentes, dans lequel les plots (34, 36) n'ont pas de matériaux incompatibles CMOS, tels que l'or.

7. Appareil selon l'une quelconque des revendications précédentes, qui comprend un appareil d'évaluation structurelle pour tester l'absence d'endommagement d'une structure.

8. Appareil selon l'une quelconque des revendications précédentes, qui comprend une membrane (24) de pas plus de 3 µm d'épaisseur s'étendant au-dessus de la chambre (26).

9. Appareil selon l'une quelconque des revendications précédentes, dans lequel la membrane mobile (24), qui se déplace pendant l'utilisation en réponse à des ultrasons incidents ou qui génère des ultrasons, a une partie centrale (28) et au moins une partie latérale qui ne se trouve pas dans le plan général de la partie centrale (28).

10. Appareil selon l'une quelconque des revendications précédentes qui possède une largeur de bande d'au moins 1 MHz (largeur de bande de 6 dB).

11. Dispositif semi-conducteur intégré ayant un appareil selon l'une quelconque des revendications 1 à 10 prévu sur un substrat semi-conducteur et un processeur de signaux ou modificateur de signaux prévu sur le même substrat semi-conducteur, intégrant le processeur et le transducteur dans le même dispositif.

12. Procédé de fabrication d'un transducteur ultrasonore consistant à appliquer une matière sacrificielle sur un substrat, à appliquer une couche polymère sur au moins une partie de la matière sacrificielle et du substrat, et éliminer au moins une partie de la matière sacrificielle pour laisser une partie de la couche polymère définissant un élément mobile, dans lequel l'élément définit une partie d'une cavité unique, et dans lequel le polymère est appliqué dans un état mobile, **caractérisé en ce que** la matière sacrificielle aide à définir une forme non plane d'une membrane polymère mobile et **en ce que** la matière sacrificielle est éliminée une fois que la forme de la membrane polymère mobile a été établie.

13. Procédé selon la revendication 12, dans lequel le revêtement de polymère qui définit l'élément mobile est appliqué sur sensiblement l'ensemble de la matière sacrificielle et est en contact avec le substrat autour de la cavité.

14. Procédé selon la revendication 12 ou la revendication 13, dans lequel le revêtement de polymère est appliqué à une certaine épaisseur de sorte à produire un élément mobile qui est de l'ordre de 2 µm d'épaisseur ou moins.

15. Procédé selon l'une quelconque des revendications 12 à 14, dans lequel le substrat est un matériau semi-conducteur.

16. Procédé selon l'une quelconque des revendications 12 à 15, consistant à appliquer une matière de plot de contact supérieure sur le dessus de la membrane, et à graver la matière à travers un masque pour définir un plot de contact supérieur.

17. Procédé selon l'une quelconque des revendications 12 à 16, consistant en outre à prévoir un dispositif à semi-conducteurs intégré ayant le transducteur et ayant les moyens de traitement de signaux prévus sur le même substrat.
